# EUROPEAN PATENT SPECIFICATION

(11) **EP 4 359 064 B1**
(45) Date of publication and mention of the grant of the patent: **02.09.2026**
(21) Application number: 22826941.1
(22) Date of filing: 24.06.2022
(51) Int. Cl.: A61N 1/36, A61N 1/04, G16H 20/30, G16H 40/67, G16H 80/00, G16H 50/20, G16H 40/63, A61N 1/372, G06F 1/16

(54) **SMART GARMENT FOR REMOTE DELIVERY OF THERAPY**
INTELLIGENTES KLEIDUNGSSTÜCK ZUR ENTFERNTEN THERAPIEVERABREICHUNG
VÊTEMENT INTELLIGENT POUR L'ADMINISTRATION À DISTANCE D'UNE THÉRAPIE

(30) Priority: 24.06.2021 US 202163214583 P
(43) Date of publication of application: 01.05.2024
(73) Proprietor: Myant Inc., Toronto, Ontario M9W 1B6 (CA)
(72) Inventor: MOINEAU, Bastien, Toronto, Ontario M9W 1B6 (CA); ALIZADEH-MEGHRAZI, Milad, Toronto, Ontario M9W 1B6 (CA); MAHNAM, Amin, Toronto, Ontario M9W 1B6 (CA)
(74) Representative: Lehmann Novo, Maria Isabel
(86) International application number: PCT/CA2022/051021
(87) International publication number: WO 2022/266775

(56) References cited:
- US-A1- 2018 036 531
- US-A1- 2018 353 365
- US-A1- 2019 110 950
- US-A1- 2020 237 031
- US-A1- 2020 367 823
- US-A1- 2020 367 823
- US-B2- 8 560 075

## Description

### FIELD

This disclosure relates to smart garments, and more particularly to smart garments for remote delivery of therapy.

### BACKGROUND

Certain forms of therapy are difficult to deliver outside of a clinical setting. For example, functional electrical stimulation (FES) is used to train or assist muscle paralysis but is mostly limited to clinical settings with well-trained therapists because of its complex set-up configuration. An exemplary system for remote delivery of therapy is disclosed in US 2020/237031 A1.

### SUMMARY

The invention provides a system according to claim 1. Embodiments of the invention are defined in the dependent claims. For better understanding of the invention, the present disclosure provides also further aspects and embodiments. In accordance with an aspect, there is disclosed a system for remote delivery of therapy. The system includes a garment comprising: a plurality of conductive fibres interlaced with a plurality of non-conductive fibres, the conductive fibres defining a plurality of signal paths; and a plurality of actuators disposed to administer therapeutic steps to a wearer of the garment. The system also includes a computing device comprising: a network interface; a signal interface in signal communication with the plurality of actuators by way of the plurality of signal paths; at least one memory storing processor-executable instructions; and at least one processor in communication with the at least one memory. The at least one processor is configured to execute the instructions to: receive a therapeutic payload data structure by way of the network interface from a remote therapy controller; process the therapeutic payload data structure to decode a therapeutic signal for actuating at least a given actuator of the plurality of actuators, the therapeutic signal reflective of a therapeutic step; activate the given actuator using the decoded therapeutic signal to administer the therapeutic step to the wearer of the garment.

In accordance with another aspect, there is disclosed a controller for remote delivery of therapy by way of a smart garment. The controller includes: a control interface; a network interface; at least one memory storing processor-executable instructions; and at least one processor in communication with the at least one memory The at least one processor is configured to execute the processor-executable instructions to: receive a control signal by way of the control interface; generate a therapeutic payload data structure upon processing the control signal, the therapeutic payload data structure comprising: an identifier of a particular actuator disposed at a pre-defined location on the smart garment, and control data for actuating the particular actuator to administer a therapeutic signal to a wearer of the smart garment; and transmit the therapeutic payload data structure by way of the network to a receiver in communication with the smart garment.

In accordance with yet another aspect, there is disclosed a method of delivering therapy remotely. The method includes providing, at a first location, a smart garment, the smart garment comprising: a plurality of conductive fibres interlaced with a plurality of non-conductive fibres, the conductive fibres defining a plurality of signal paths; and a plurality of actuators disposed to contact a wearer when the garment is worn; providing, at a second location, a controller, the controller comprising: an interface for receiving a signal for controlling a given actuator of the plurality of actuators; transmitting the signal from the second location to the first location by way of a network; and administering the signal to a wearer of the smart garment by way of the given actuator.

Many further features and combinations thereof concerning embodiments described herein will appear to those skilled in the art following a reading of the instant disclosure.

### BRIEF DESCRIPTION OF DRAWINGS

In the figures,
**FIG. 1** is a schematic diagram of a networked therapy sender system and therapy receiver system including a smart garment, in accordance with an embodiment;
**FIG. 2** is a schematic diagram of the smart garment of **FIG. 1****,** in accordance with an embodiment;
**FIG. 3** shows a smart garment for FES stimulation, in accordance with an embodiment;
**FIG. 4** is a flowchart showing example operations for delivering remote therapy, in accordance with an embodiment;
**FIG. 5** is a schematic diagram showing data transmission between a first location and a second location during delivery of remote therapy;
**FIG. 6** is a schematic diagram of a networked therapy sender system and therapy receiver system including a smart garment, in accordance with another embodiment; and
**FIG. 7A** shows a smart garment of the therapy sender system of **FIG. 6****,** in accordance with an embodiment;
**FIG. 7B** shows a smart garment of the therapy sender system of **FIG. 6****,** in accordance with an embodiment;
**FIG. 8** is a schematic diagram of a computing device for implementing a therapy sender system and/or a therapy receiver system, in accordance with an embodiment.

### DETAILED DESCRIPTION

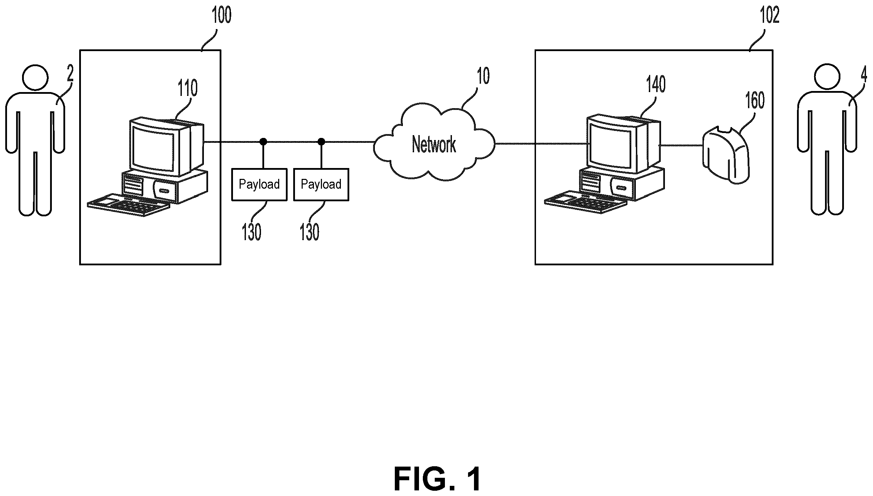

**FIG. 1** is a schematic diagram of a therapy sender system 100 and a therapy receiver system 102, in accordance with an embodiment. System 100 and system 102 cooperate in manners disclosed herein to facilitate the remote delivery of therapy using a smart garment.

Therapy sender system 100 is disposed at a first location while therapy receiver system 102 is disposed at a second location remote from the first location. Therapy sender system 100 and therapy receiver system 102 are interconnected by way of communication network 10, which facilitates data communication between the two systems. As detailed herein, therapy sender system 100 generates therapeutic signals, and sends such signals across network 10 to therapy receiver system 102. In some embodiments, a therapeutic signal may include data and/or instructions for therapeutic steps to be performed at receiver system 102.

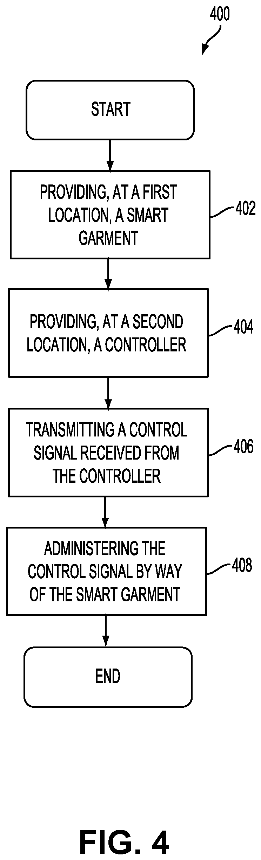

Therapy sender system 100 includes a computing device 110 that controls system 100 to generate therapeutic signals. For brevity, computing device 110 may also be referred to herein as controller 110. In the depicted embodiment, an operator 2 of controller 110 provides user input and controller 110 generates therapeutic signals in accordance with this user input. Operator 2 may be a caregiver such as a therapist, physician, nurse, other medical professional, or the like. Controller 110 receives the user input from operator 2 through a user interface or other type of I/O interface (as shown in **FIG. 4****).** In some embodiments, this user interface may, for example, include a keyboard, mouse, touchscreen or other type of input interface. In some embodiments, controller 110 may generate therapeutic signals in accordance with data stored in a non-volatile memory. In some embodiments, controller 110 may generate therapeutic signals in accordance with a pre-defined algorithm, a trained neural network, or the like.

Therapeutic signals are encoded at controller 110 for transmission across network 10 to therapy receiver system 102. For example, controller 110 may generate one or more therapeutic payload data structures 130 including the therapeutic signals. For example, a payload data structure 130 may include an identifier of a particular actuator disposed at a pre-defined location on smart garment 160, and control data for actuating that particular actuator to administer a therapeutic signal to a wearer of smart garment 160.

In some embodiments, a therapeutic payload data structure 130 may be transmitted in the form of a data stream from controller 110 to therapy receiver system 102. Such data streams may provide therapeutic signals such that therapeutic steps can be implemented in real time or near real time at therapy receiver system 102.

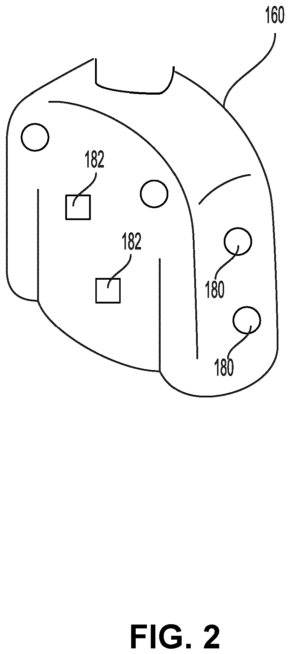

Therapy receiver system 102 includes a receiver device 140 and a smart garment 160, which may be worn by a patient 4. As best seen in **FIG. 2****,** smart garment 160 includes a plurality of actuators 180 which may be activated by the therapeutic signals to administer therapeutic steps to patient 4.

Receiver device 140 receives one or more therapeutic data structures 130 from controller 110 by way of network 10. Receiver device 140 processes a data structure 130 to decode the therapeutic signal and obtain (i) identifier(s) of at least one particular actuator 180 disposed at a pre-defined location or locations on smart garment 160 and (ii) control data for actuating the at least one particular actuator 180 to administer the therapeutic signal to a wearer of smart garment 160. Receiver device 140 transmit the decoded signal to smart garment 160 to activate the at least one particular actuator 180, and thereby administer the therapeutic step to patient 4.

In some embodiments, therapy receiver system 102 provides feedback to therapy sender system 100, e.g., to provide information regarding how patient 4 is responding to administered therapy, under control of therapy sender system 100. In such embodiments, smart garment 160 also includes a plurality of sensors 182 for sensing a state of patient 4 and/or a state of garment 160.

In such embodiments, sensor signals are encoded at receiver device 140 for transmission across network 10 to therapy sender system 102. For example, receiver device 140 may generate one or more feedback data structures including a sensor signal, such as, for example, an electrophysiological signal or a biomechanical signal. In some embodiments, a feedback data structure may include an identifier of the particular sensor that sensed a given sensor signal. In some embodiments, a feedback data structure may be transmitted in the form of a data stream from receiver device 140 to therapy sender system 100. Such data streams may provide sensor signals such that therapy sender system 100 can respond (e.g., by adapting therapeutic steps) in real time or near real time.

Controller 110 receives a feedback data structure and processes it to decode a sensor signal. In some embodiments, controller 110 may display the sensor signal to operator 2. In some embodiments, controller 110 may automatically adapt subsequent therapeutic steps in response to the sensor signal. In some embodiments, controller 110 may generate an alert signal in response to the sensor signal, e.g., to notify operator 2 of a state of patient 4 or a state of garment 160.

Actuators 180 may include various types of actuators suitable for applying current/voltage to patient 4 for Functional Electrical Stimulation (FES), Transcranial Current Stimulation (TCS), Transcutaneous electrical nerve stimulation (TENS), HighFrequency Alternating Current Stimulation, and/or creating a tactile sensation. Actuators 180 may also include actuators suitable for providing temperature regulation (e.g., heaters to provide heating or coolers to provide cooling). Actuators 180 may also include actuators suitable dispensing medication, e.g., medication for providing localized pain relief, for promoting wound healing, etc. Actuators 180 may include actuators suitable changing the permeability of the skin, e.g., through iontophoresis (e.g., including electrophoresis and/or electroosmosis), to facilitate transdermal delivery of medication Actuators 180 may include actuators suitable for imparting mechanical vibration (e.g. vibrating the air or vibrating the skin atop a target body part). For example, actuator 180 may include a haptic actuator.

Sensors 182 may include various types of sensors suitable for sensing electrophysiological signals including Electromyogram (EMG), Electroencephalogram (EEG), Electrocardiogram (ECG), Electrooculogram (EOG), and Electrogastrogram (EGG) signals, or the like. Sensors 182 may also include various sensors suitable for sensing biomechanical feedback such as stretch sensors, pressure sensors, accelerometers, gyroscopes, magnetometer, inertial measurement units, or the like. Sensors 182 may also include sensors suitable sensing body temperature, blood pressure, pulse, etc. Sensors 182 may also include sensors suitable for sensing the presence of bodily fluids such as sweat, blood, urine, etc.

In some embodiments, one or more of actuators 180 and sensors 182 may be formed of an electrode acting as a transducer in converting the ionic current in/on the body into electron currents in conductive wires and electronic circuits, and vice versa. An electrode may generally be defined as conductive material through which electricity passes to a body of a user and/or is received from the body of a user. An electrode can function as a sensor when receiving electrical energy for measurement/recordation. An electrode can function as an actuator when injecting electrical current/voltage to the body, e.g. for FES to inject electrical pulses to activate muscles.

In the depicted embodiments, one or more of actuators 180 and sensors 182 may be formed of a dry contact electrode. Dry contact electrodes can be categorized according to form factor into textile electrodes, flexible film electrodes, bulk electrodes, pin-shaped electrodes, and microneedles. Dry electrodes may be biocompatible, easy to use, comfortable, breathable, lightweight, flexible, washable, durable, and able to maintain good signal quality during electrophysiology testing while at rest and moving. Additionally, textile-based electrodes may be worn on various body parts by attaching them to different articles of clothing such as waistbands, sleeves, pants, headbands, etc.

Dry contact electrodes may be more convenient than standard wet gel electrodes in some respects. For example, standard electrodes may use an electrolytic gel to maintain good electrical contact with the Stratum Corneum, creating an ionic path between the electrode and the skin below the Stratum Corneum via conductive ions in the gel. This reduces the skin impedance and allows for improved signal acquisition. However, the standard wet gel electrode used currently, e.g. in healthcare, may have limitations. The adhesive can cause skin irritation and becomes uncomfortable over time, the gel dehydrates with time thus degrading signal quality, and the electrode can be uncomfortable to the user, due to its metallic piece, therefore a soft, textile form is an inconspicuous alternative for continuous health monitoring.

In the depicted embodiment, one or more of actuators 180 and sensors 182 may be a textile-based electrode. Such an electrode may include conductive fibres interlaced with the non-conductive fibres. Such an electrode may be integrally formed with smart garment 160.

In some embodiments, one or more of actuators 180 and sensors 182 may be a dry contact, textile-based electrode, as disclosed for example in PCT Patent Application No. PCT/CA2020/051809 , entitled "CONDUCTIVE THERMOPLASTIC ELASTOMER ELECTRODES, AND METHOD OF MANUFACTURING SUCH ELECTRODES", the entire contents of which are herein incorporated by reference.

**FIG. 2** schematically illustrates a smart garment 160, in accordance with an embodiment. As depicted, smart garment 160 is adapted to be disposed over an upper body section of an individual, such as a t-shirt, a long-sleeved shirt, a blouse, a dress, among others. In this embodiment, actuators 180 and sensors 182 may be disposed in smart garment 160 to be positioned in relation to (and optionally contact) particular body regions of a patient 4, e.g., as sleeves or cuffs around the biceps, triceps, chest, wrists, etc.

In another embodiment, smart garment 160 may be adapted to be disposed over a lower body section of an individual, such as a pair of pants, shorts, underwear, socks, among others. In this embodiment, actuators 180 and sensors 182 may be disposed in smart garment 160 to be positioned in relation to (and optionally contact) other particular body regions of a patient 4, as cuffs or pant legs around the ankles, calves, thighs, etc.

In another embodiment, smart garment 160 may be disposed over another body section of an individual such as a head or one or more of the extremities (e.g., a hand or foot).

In another embodiment, smart garment 160 may be disposed over multiple body sections of an individual.

In the depicted embodiment, smart garment 160 is formed of a knitted textile. Smart garment 160 includes a plurality of conductive fibres interlaced with a plurality of non-conductive fibres. The conductive fibres define a plurality of signal paths suitable for delivering data and/or power to actuators 180 and sensors 182.

In some embodiments, smart garment 160 may be formed of other textile forms and/or techniques such as weaving, knitting (warp, weft, etc.) or the like. In some embodiments, smart garment 160 includes any one of a knitted textile, a woven textile, a cut and sewn textile, a knitted fabric, a non-knitted fabric, in any combination and/or permutation thereof. Example structures and interlacing techniques of textiles formed by knitting and weaving are disclosed in U.S. Patent Application No. US15/267,818, entitled "Conductive Knit Patch", the entire contents of which are herein incorporated by reference.

As used herein, "textile" refers to any material made or formed by manipulating natural or artificial fibres to interlace to create an organized network of fibres. Generally, textiles are formed using yarn, where yarn refers to a long continuous length of a plurality of fibres that have been interlocked (i.e. fitting into each other, as if twined together, or twisted together). Herein, the terms fibre and yarn are used interchangeably. Fibres or yarns can be manipulated to form a textile according to any method that provides an interlaced organized network of fibres, including but not limited to weaving, knitting, sew and cut, crocheting, knotting and felting.

Different sections of a textile can be integrally formed into a layer to utilize different structural properties of different types of fibres. For example, conductive fibres can be manipulated to form networks of conductive fibres and non-conductive fibres can be manipulated to form networks of non-conductive fibers. These networks of fibres can comprise different sections of a textile by integrating the networks of fibres into a layer of the textile. The networks of conductive fibres can form one or more conductive pathways that electrically connect with actuators 180 and sensors 182 embedded in smart garment 160, for conveying data and/or power to and/or from these components.

In some embodiments, multiple layers of textile can also be stacked upon each other to provide a multi-layer textile.

As used herein, "interlace" refers to fibres (either artificial or natural) crossing over and/or under one another in an organized fashion, typically alternately over and under one another, in a layer. When interlaced, adjacent fibres touch each other at intersection points (e.g. points where one fibre crosses over or under another fibre). In one example, first fibres extending in a first direction can be interlaced with second fibres extending laterally or transverse to the fibres extending in the first connection. In another example, the second fibres can extend laterally at 90° from the first fibres when interlaced with the first fibres. Interlaced fibres extending in a sheet can be referred to as a network of fibres.

As used herein "integrated" or "integrally" refers to combining, coordinating or otherwise bringing together separate elements so as to provide a harmonious, consistent, interrelated whole. In the context of a textile, a textile can have various sections comprising networks of fibres with different structural properties. For example, a textile can have a section comprising a network of conductive fibres and a section comprising a network of non-conductive fibres. Two or more sections comprising networks of fibres are said to be "integrated" together into a textile (or "integrally formed") when at least one fibre of one network is interlaced with at least one fibre of the other network such that the two networks form a layer of the textile. Further, when integrated, two sections of a textile can also be described as being substantially inseparable from the textile. Here, "substantially inseparable" refers to the notion that separation of the sections of the textile from each other results in disassembly or destruction of the textile itself.

In some examples, conductive fabric (e.g. group of conductive fibres) can be knit along with (e.g. to be integral with) the base fabric (e.g. surface) in a layer. Such knitting may be performed using a circular knit machine or a flat bed knit machine, or the like, from a vendor such as Santoni or Stoll.

Conveniently, cooperation of therapy sender system 100 and therapy receiver system 102 allows therapy to be delivered to a first location from a second location that is remote from the first location.

### Functional Electrical Stimulation (FES) Example

The prevalence of stroke is about 1,900 cases per 100,000 individuals and its yearly incidence about 250 per 100,000²⁰. Up to 85% of individuals who had a stroke have initial paralysis in the arm, and 55 to 75% still have upper limb impairments 3 to 6 months following the stroke¹⁶. As for SCI, the prevalence is about 80 individuals per 100,000 and the yearly incidence about 4 per 100,000^{21,29}. Within those with tetraplegia (i.e. cervical SCI, 54% of SCI at discharge), paralysis-related hand dysfunction is the major concern⁶.

Rehabilitation can enhance neurological and functional recovery after stroke^{3,18} and SCI^{9,22,23}. Among the rehabilitation modalities is functional electrical stimulation (FES)^{10,11}, a therapeutic technique applying sequences of pulses to the peripheral nerves to produce muscle contractions in functional patterns²⁴. Repeated use of FES has been shown to improve voluntary command, muscle and bone composition, and function after stroke^{5,14,30} and SCI^{2,7,8,17}. Conventionally, FES is administered via adhesive disposable gel electrodes secured on the skin with adhesive tape or wrap. This setup requires knowledge of where to place the electrodes and bimanual dexterity, which is limited in most individuals with paralysis due to stroke or cervical SCI. Thus, while multiple sessions are necessary to yield clinical results, FES cannot be applied independently and is limited to a clinical setting. Moreover, the lack of resources and difficulty of use of FES systems deters therapists from using FES in rehabilitation and end-users to receive home- or community-based FES interventions^{1,26}.

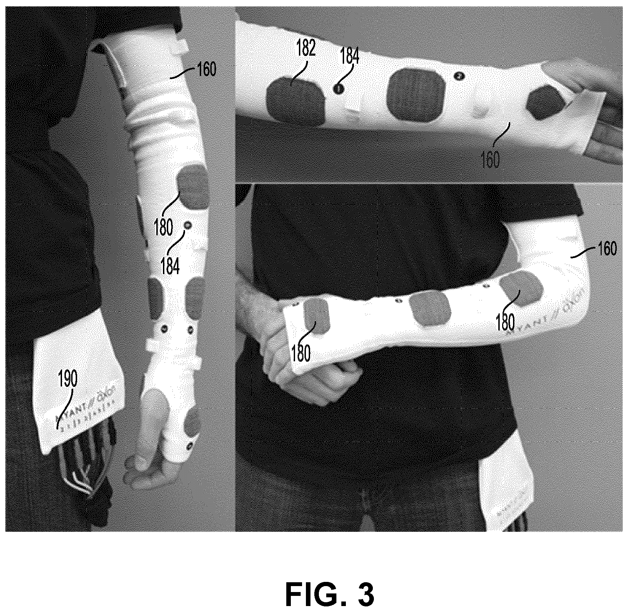

**FIG. 3** depicts an embodiment of smart garment 160, adapted for administering FES therapy. For convenience, this embodiment of smart garment 160 may be referred to as FES shirt 160. FES shirt 160 may be used as part of a system for delivering remote therapy to patients who have upper-limb paralysis due to stroke or spinal-cord injury.

FES shirt 160 includes a plurality of actuators 180 in the form of textile-based electrodes at the sites of stimulation. The electrodes are integrally formed with FES shirt 160, and are pre-positioned to contact targeted body portions. The electrodes facilitate simultaneous or sequential stimulation on different muscles, e.g., under the control of therapy sender system 100. The electrodes may be disposed on FES shirt 160, for example, to stimulate finger extensors and flexors, abductor/opposition muscles, lumbricals, flexor digitorum muscles; shoulder muscles, elbow muscles, finger muscles, thumb abductor, deltoids, biceps, triceps, etc. In other embodiments, other muscles (e.g., lower body muscles) may be similarly stimulated.

In the depicted embodiment, FES shirt 160 includes a tight elastic arm-length sleeve, assembled from flat panels of conductive and non-conductive yarns produced by a knitting machine, e.g., a flat-bed knitting machine from a vendor such as Stoll. The conductive and non-conductive yarns are interlaced to form a plurality of signal paths which are interconnected with actuators 180.

Actuators 180 are in electrical communication with a connector 190 by way of the signal paths. Connector 190 is connected to a stimulator that generates FES signals under control of receiver device 140. The stimulator may, for example, be configured to deliver 300µs square balanced asymmetrical pulses at 40 Hz. In some embodiments, pulse current may for example be approximately 10-30 mA, or above. Each channel may be used to stimulate a corresponding muscle group. For example, in the case of a 4-channel stimulator, each channel may be assigned to stimulate a corresponding one of the following muscle groups: (i) flexor digitorium superficialis and profundus, (ii), thumb opponent and abductors, (iii) lumbrical muscles, and (iv) extensor digitorium.

In the depicted embodiment, the sleeves of FES shirt 160 are formed of thick double-layer textile with electrodes that are raised for improving conformity with the skin Such raised electrodes have a higher contact pressure than non-raised areas.

In some embodiments, FES shirt 160 includes a full front trunk zipper to ease donning. In some embodiments, FES shirt 160 may include forearm zippers to ease donning. In some embodiments, FES shirt 160 may include a tightening band across the front of the chest to secure the deltoid electrodes' positions. This band may include a magnetic clip to ease donning.

In some embodiments, an actuator 180 may be formed of an electrode having hydrophobic yarn on the outer surface of smart garment 160 to maintain wetness longer.

In some embodiments, an actuator 180 may be formed of an electrode having electrically non-conductive yarn on the outer surface of smart garment 160 to reduce sensation of electricity upon contact therewith.

In some embodiments, FES shirt 160 includes an identification numeral 184 beside each electrode, which matches corresponding numbers on connector 190 to assist in electrode identification.

During wear, water may be applied to a skin-contacting surface of the electrodes, e.g., by a spray bottle to improve signal transmission.

Conveniently, some embodiments of FES shirt 160 may reduce the time and complexity of FES setup. Conveniently, some embodiments of FES shirt 160 may be used outside of a clinical setting. Conveniently, some embodiments of FES shirt 160 may be used by individuals independently capable of upper-body dressing.

Optionally, a fitting session may be used to calibrate positions for the electrodes of FES shirt 160 for a particular patient 4. In an example fitting session, desired FES electrode positions are identified using gel electrodes (or another type of electrode), a canvas shirt is donned over the electrodes, the canvas shirt is adjusted with safety pins, and electrode positions are drawn on the canvas shirt. The canvas shirt is used to manufacture FES shirt 160 with integrated electrodes are the calibrated positions. Alternatively, other methods may be used to manually or automatically customize FES shirt 160 to a particular patient 4, including the locations of integrated electrodes.

### References

1. Auchstaetter, N., J. Luc, S. Lukye, K. Lynd, S. Schemenauer, M. Whittaker, and K. E. Musselman. Physical Therapists' Use of Functional Electrical Stimulation for Clients With Stroke: Frequency, Barriers, and Facilitators. Phys. Ther. 96:995-1005, 2016.
2. Baldi, J. C., R. D. Jackson, R. Moraille, and W. J. Mysiw. Muscle atrophy is prevented in patients with acute spinal cord injury using functional electrical stimulation. Spinal Cord 36:463-9, 1998.
3. Bernhardt, J., E. Godecke, L. Johnson, and P. Langhorne. Early rehabilitation after stroke. Curr. Opin. Neurol. 30:48-54, 2017.
4. Creswell, J. W., M. D. Fetters, V. L. Plano Clark, and A. Morales. Mixed Methods Intervention Trials. In: Mixed Methods Research for Nursing and the Health Sciences. Oxford, UK: Wiley-Blackwell, 2009, pp. 159-180.doi:10.1002/9781444316490.ch9
5. Daly, J. J., J. Zimbelman, K. L. Roenigk, J. P. McCabe, J. M. Rogers, K. Butler, R. Burdsall, J. P. Holcomb, E. B. Marsolais, and R. L. Ruff. Recovery of Coordinated Gait: Randomized Controlled Stroke Trial of Functional Electrical Stimulation (FES) Versus No FES, With Weight-Supported Treadmill and Over-Ground Training. Neurorehabil. Neural Repair 25:588-596, 2011.
6. French, J. S., K. D. Anderson-Erisman, and M. Sutter. What do spinal cord injury consumers want? A review of spinal cord injury consumer priorities and neuroprosthesis from the 2008 neural interfaces conference. Neuromodulation 13:229-231, 2010.
7. Frotzler, A., S. Coupaud, C. Perret, T. H. Kakebeeke, K. J. Hunt, N. de N. Donaldson, and P. Eser. High-volume FES-cycling partially reverses bone loss in people with chronic spinal cord injury. Bone 43:169-176, 2008.
8. Giangregorio, L., C. Craven, K. Richards, N. Kapadia, S. L. Hitzig, K. Masani, and M. R. Popovic. A randomized trial of functional electrical stimulation for walking in incomplete spinal cord injury: effects on body composition. J. Spinal Cord Med. 35:351-60, 2012.
9. de Groot, P. C. E., N. Hjeltnes, A. C. Heijboer, W. Stal, and K. Birkeland. Effect of training intensity on physical capacity, lipid profile and insulin sensitivity in early rehabilitation of spinal cord injured individuals. Spinal Cord 41:673-9, 2003.
10. Ho, C. H., R. J. Triolo, A. L. Elias, K. L. Kilgore, A. F. DiMarco, K. Bogie, A. H. Vette, M. L. Audu, R. Kobetic, S. R. Chang, K. M. Chan, S. Dukelow, D. J. Bourbeau, S. W. Brose, K. J. Gustafson, Z. H. T. Kiss, and V. K. Mushahwar. Functional electrical stimulation and spinal cord injury. Phys. Med. Rehabil. Clin. N. Am. 25:631-54, ix, 2014.
11. Howlett, O. A., N. A. Lannin, L. Ada, and C. Mckinstry. Functional electrical stimulation improves activity after stroke: A systematic review with meta-analysis. Arch. Phys. Med. Rehabil. 96:934-943, 2015.
12. Itzkovich, M., I. Gelernter, F. Biering-Sorensen, C. Weeks, M. T. Laramee, B. C. Craven, M. Tonack, S. L. Hitzig, E. Glaser, G. Zeilig, S. Aito, G. Scivoletto, M. Mecci, R. J. Chadwick, W. S. El Masry, A. Osman, C. A. Glass, P. Silva, B. M. Soni, B. P. Gardner, G. Savic, E. M. Bergström, V. Bluvshtein, J. Ronen, and A. Catz. The Spinal Cord Independence Measure (SCIM) version III: Reliability and validity in a multi-center international study. Disabil. Rehabil. 29:1926-1933, 2007.
13. Kapadia, N., V. Zivanovic, M. Verrier, and M. Popovic. Toronto Rehabilitation Institute-Hand Function Test: Assessment of Gross Motor Function in Individuals With Spinal Cord Injury. Top. Spinal Cord Inj. Rehabil. 18:167-186, 2012.
14. Kawashima, N., M. R. Popovic, and V. Zivanovic. Effect of intensive functional electrical stimulation therapy on upper-limb motor recovery after stroke: Case study of a patient with chronic stroke. Physiother. Canada 65:20-28, 2013.
15. Keith, R., C. Granger, B. Hamilton, and F. Sherwin. The functional independence measure: a new tool for rehabilitation. Adv. Clin. Rehabil. 1:6-18, 1987.
16. Lai, S. M., S. Studenski, P. W. Duncan, and S. Perera. Persisting consequences of stroke measured by the stroke impact scale. Stroke 33:1840-1844, 2002.
17. Martin, R., C. Sadowsky, K. Obst, B. Meyer, and J. McDonald. Functional electrical stimulation in spinal cord injury: from theory to practice. Top. Spinal Cord Inj. Rehabil. 18:28-33, 2012.
18. Maulden, S. A., J. Gassaway, S. D. Horn, R. J. Smout, and G. DeJong. Timing of initiation of rehabilitation after stroke. Arch. Phys. Med. Rehabil. 86:34-40, 2005.
19. Moineau, B., C. Marquez-Chin, M. Alizadeh-meghrazi, and M. R. Popovic. Garments for functional electrical stimulation : Design and proofs of concept. J. Rehabil. Assist. Technol. Eng. Accepted:, 2019.
20. Mozaffarian, D. et al. Heart Disease and Stroke Statistics-2016 Update. Circulation 133:e38-e48, 2016.
21. National Spinal Cord Injury Statistical Center. 2014 Annual Statistical Report Complete Public Version. 2014.
22. Panisset, M. G., M. P. Galea, and D. El-Ansary. Does early exercise attenuate muscle atrophy or bone loss after spinal cord injury? Spinal Cord 54:1-9, 2016.
23. Scivoletto, G., B. Morganti, and M. Molinari. Early versus delayed inpatient spinal cord injury rehabilitation: An Italian study. Arch. Phys. Med. Rehabil. 86:512-516, 2005.
24. Sheffler, L. R., and J. Chae. Neuromuscular electrical stimulation in neurorehabilitation. Muscle and Nerve 35:562-590, 2007.
25. Smith, C., L. Kenney, D. Howard, K. Waring, M. Sun, H. Luckie, N. Hardiker, and S. Cotterill. Prediction of setup times for an advanced upper limb functional electrical stimulation system. J. Rehabil. Assist. Technol. Eng. 5:205566831880256, 2018.
26. Taylor, M. J., A. J. Ruys, C. Fornusek, M. Bijak, M. Russold, and A. E. Bauman. Lessons from Vienna: stakeholder perceptions of functional electrical stimulation technology and a conceptual model for practice. Disabil. Rehabil. Assist. Technol. 0:1-8, 2018.
27. Wilkinson, C. R., and A. De Angeli. Applying user centred and participatory design approaches to commercial product development. Des. Stud. 35:614-631, 2014.
28. Wolf, S. L., P. A. Catlin, M. Ellis, A. L. Archer, B. Morgan, and A. Piacentino. Assessing Wolf Motor Function Test as outcome measure for research in patients after stroke. Stroke 32:1635-1639, 2001.
29. Wyndaele, M., and J.-J. Wyndaele. Incidence, prevalence and epidemiology of spinal cord injury: what learns a worldwide literature survey? Spinal Cord 44:523-529, 2006.
30. Yan, T., C. W. Y. Hui-Chan, and L. S. W. Li. Functional electrical stimulation improves motor recovery of the lower extremity and walking ability of subjects with first acute stroke: A randomized placebo-controlled trial. Stroke 36:80-85, 2005.

### Example Operation

**FIG. 4** depicts example operation for delivering remote therapy using smart garment 160, in accordance with an embodiment. In particular, **FIG. 4** shows blocks 400 and onward, which are performed at therapy sender system 100 and therapy receiver system 102, in accordance with an embodiment.

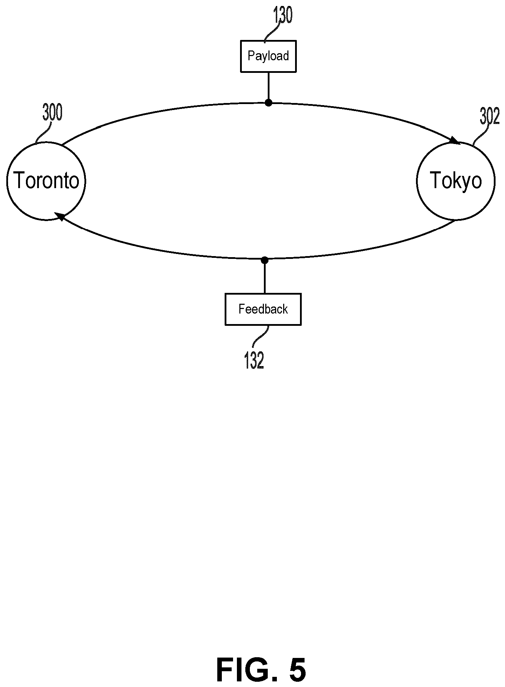

At block 402, a therapy receiver system 102 with a smart garment 160 is provided a first location 302 **(****FIG. 5****).** Smart garment 160 is worn by a patient 4.

At block 404, a therapy sender system 102 with a controller 110 is provided at a second location 300 **(****FIG. 5****).** Second location 300 may be remote from first location 302. In one specific example shown in **FIG. 5****,** first location 302 is in Tokyo while second location 300 is in Toronto. Controller 110 includes a control interface operable by operator 2, and receives signals for controlling actuators of smart garment 160 by way of this control interface. Such signals may be referred to as control signals or therapeutic signals. These signals are encoded in payload data structures 130 for transmission from second location 300 to first location 302.

At block 406, one or more payload data structures (encoding the control signals) are transmitted to second location 300, where they are received at receiver device 140. Receiver device 140 decodes the control signal.

At block 408, receiver device 140 uses the decoded signal to activate a particular actuator 180 (as identified in the signal) of smart garment 160, and thereby administer a therapeutic step to patient 4.

In some embodiments, receiver device 400 may receive sensor signals from sensors 182 of smart garment 160 Receiver device 400 encodes the signal sensors in feedback data structures 132 that are transmitted from first location 302 to second location 300.

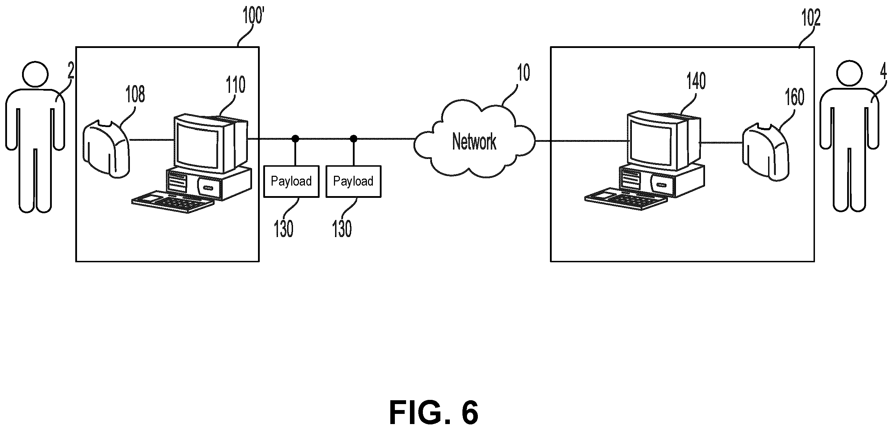

**FIG. 6** a schematic diagram of a therapy sender system 100' and a therapy receiver system 102, in accordance with an embodiment.

Therapy sender system 100' includes a smart garment 108 that is worn by operator 2 to receive user input. For example, smart garment 108 includes a plurality of sensors 182 that measure muscle activity of operator 2. For example, one or more of sensors 182 may be an EMG sensor for measuring EMG signals. Smart garment 108 may be otherwise substantially similar to garment 160. The EMG signals sensed from operator 2 are processed at controller 110 to generate corresponding FES signals for stimulating patient 4. As an example, controller 110 may process EMG signals obtained via smart garment 108 to recognize a pre-defined pattern A as an indicator that generation of a pre-defined FES signal B should be initiated or interrupted. A mapping table or similar data structure may be used to establish mappings between pre-defined patterns A and pre-defined FES signals B. In some embodiments, such mappings may be customized for a particular operator 2 and/or a particular participant 4. In some embodiments, controller 110 may implement known or other pattern recognition or machine learning techniques to detect the presence of a pattern A in EMG signals.

The generated FES signals are encoded and transmitted as a portion of the therapeutic signals described herein. The FES signals are decoded by receiver device 140 and used to activate FES actuators 180 of garment 160. In this way muscle movements made by an operator 2 can be recorded at therapy sender system 100' and replicated on patient 4 at therapy receiver system 102 as part of delivering therapy.

In some embodiments, controller 110 may receive information regarding a clinically relevant condition or event (e.g., completion or non-completion of a task by patient 4) from therapy receiver system 102, e.g., by way of a signal transmitted across network 10. In such embodiments, smart garment 108 may include one or more actuators 180. An actuator 180 may be activated in response to receiving a signal of a clinically relevant condition or event, and thereby provide an alert to operator 2 corresponding to the clinically relevant condition or event. The actuator 180 may, for example, include a haptic actuator which imparts a mechanical vibration to generate the alert.

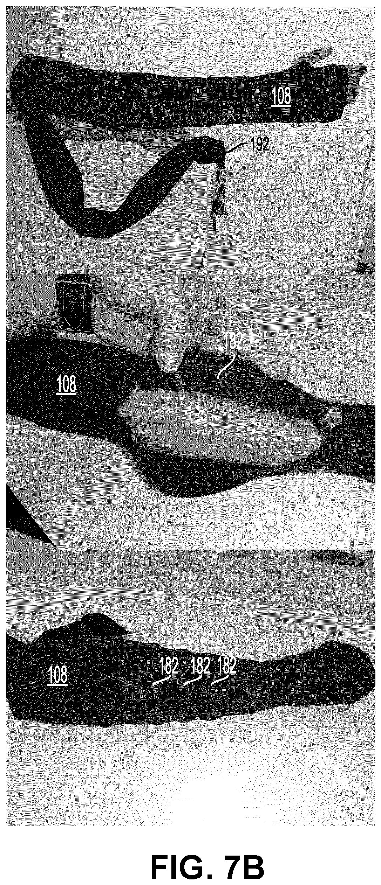

Smart garment 108 may be connected to controller 110 by way of a connector 192 **(****FIG. 7B****),** which establish electrical communication between components of smart garment 108 (e.g., sensors 182 and/or actuators 180) and controller 110. Such electrical communication allows, for example, signals to be transmitted from a sensor 182 to controller 110, and for signals to be transmitted from controller 110 to an actuator 180.

In some embodiments, therapy sender system 100' may be operated with patient 4 wearing smart garment 108 and operator 2 wearing smart garment 160. When operated in this way, operator 2 can receive information regarding muscle activation of a patient 4 (e.g., as measured via EMG signals) which may include clinically-relevant information. The information may be received by operator 2 via actuators 180 of smart garment 160, e.g., in the form of FES, mechanical vibrations, or the like.

Therapy sender system 100' is otherwise substantially similar to therapy sender system 100.

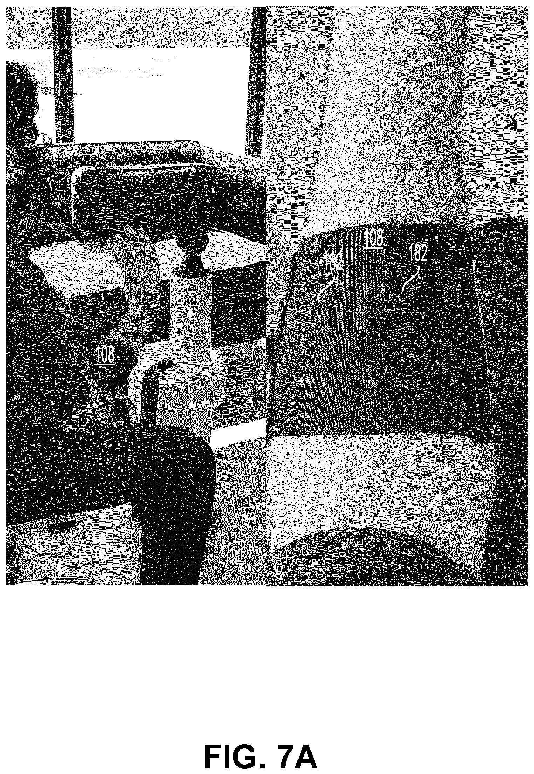

**FIG. 7A** shows smart garment 108, in accordance with an embodiment. In this embodiment, smart garment 108 is worn on a portion of the forearm of operator 2.

**FIG. 7B** shows smart garment 108, in accordance with another embodiment. In this embodiment, smart garment 108 extends a length of the arm of operator 2 from above the elbow to the hand.

**FIG. 7A** and **7B** each show textile electrodes functioning as sensors 182.

Embodiments have been described herein with reference to a therapy sender system 100 is disposed at a first location while therapy receiver system 102 is disposed at a second location remote from the first location. However, in other embodiments, therapy sender system 100 and therapy receiver system 102 may be located at the same location. For example, operator 2 and patient 4 may be at the same location, which may be, for example, the patient's home, a clinical facility, or the like.

### Additional Implementation Details

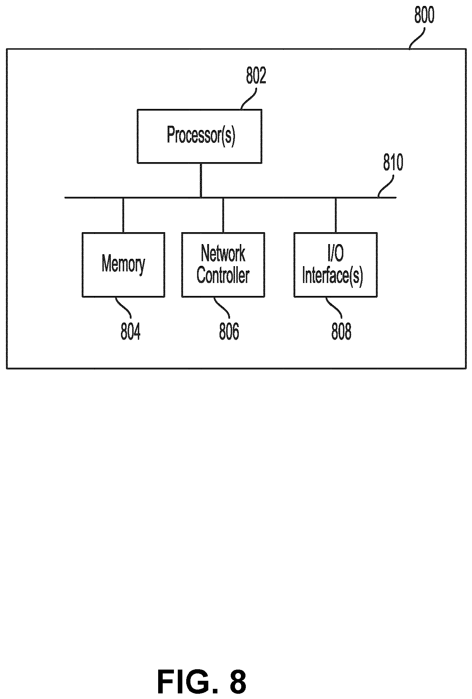

**FIG. 8** is a schematic diagram of computing device 800 which may be used to implement one or both of controller 110 and receiver device 140, exemplary of an embodiment. As depicted, computing device 800 includes at least one processor 802, memory 804, at least one I/O interface 806, and at least one network interface 808, which may be interconnected by a bus 810.

Each processor 802 may be, for example, any type of general-purpose microprocessor or microcontroller, a digital signal processing (DSP) processor, an integrated circuit, a field programmable gate array (FPGA), a reconfigurable processor, a programmable read-only memory (PROM), or any combination thereof.

Memory 804 may include a suitable combination of any type of computer memory that is located either internally or externally such as, for example, random-access memory (RAM), read-only memory (ROM), compact disc read-only memory (CDROM), electro-optical memory, magneto-optical memory, erasable programmable read-only memory (EPROM), and electrically-erasable programmable read-only memory (EEPROM), Ferroelectric RAM (FRAM) or the like.

Each I/O interface 806 enables controller 110 and/or receiver device 140 to interconnect with one or more input devices, such as a keyboard, mouse, camera, touch screen and a microphone, or with one or more output devices such as a display screen and a speaker. In the embodiment depicted in **FIG. 6****,** an I/O interface 806 enables controller 110 to interconnect with smart garment 108 and receive input therefrom.

Each network interface 808 enables controller 110 and/or receiver device 140 to communicate with other components, to exchange data with other components, to access and connect to network resources, to serve applications, and perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others, including any combination of these.

For simplicity only, one computing device 800 is shown but system 110 and/or system 102 may include multiple computing devices 800. The computing devices 800 may be the same or different types of devices. The computing devices 800 may be connected in various ways including directly coupled, indirectly coupled via a network, and distributed over a wide geographic area and connected via a network (which may be referred to as "cloud computing").

For example, and without limitation, a computing device 800 may be a server, network appliance, set-top box, embedded device, computer expansion module, personal computer, laptop, personal data assistant, cellular telephone, smartphone device, UMPC tablets, video display terminal, gaming console, or any other computing device capable of being configured to carry out the methods described herein.

The foregoing discussion provides many example embodiments. Although each embodiment represents a single combination of inventive elements, other examples may include all possible combinations of the disclosed elements. Thus if one embodiment comprises elements A, B, and C, and a second embodiment comprises elements B and D, other remaining combinations of A, B, C, or D, may also be used.

The term "connected" or "coupled to" may include both direct coupling (in which two elements that are coupled to each other contact each other) and indirect coupling (in which at least one additional element is located between the two elements).

The technical solution of embodiments may be in the form of a software product. The software product may be stored in a non-volatile or non-transitory storage medium, which can be a compact disk read-only memory (CD-ROM), a USB flash disk, or a removable hard disk. The software product includes a number of instructions that enable a computer device (personal computer, server, or network device) to execute the methods provided by the embodiments.

The embodiments described herein are implemented by physical computer hardware, including computing devices, servers, receivers, transmitters, processors, memory, displays, and networks. The embodiments described herein provide useful physical machines and particularly configured computer hardware arrangements. The embodiments described herein are directed to electronic machines and methods implemented by electronic machines adapted for processing and transforming electromagnetic signals which represent various types of information. The embodiments described herein pervasively and integrally relate to machines, and their uses; and the embodiments described herein have no meaning or practical applicability outside their use with computer hardware, machines, and various hardware components. Substituting the physical hardware particularly configured to implement various acts for non-physical hardware, using mental steps for example, may substantially affect the way the embodiments work. Such computer hardware limitations are clearly essential elements of the embodiments described herein, and they cannot be omitted or substituted for mental means without having a material effect on the operation and structure of the embodiments described herein. The computer hardware is essential to implement the various embodiments described herein and is not merely used to perform steps expeditiously and in an efficient manner.

Although the embodiments have been described in detail, it should be understood that various changes, substitutions and alterations which fall under the scope of the claims, may be made.

As can be understood, the examples described above and illustrated are intended to be exemplary only. The scope is indicated by the appended claims.

## Claims

1. A system for remote delivery of therapy, the system comprising:
a first garment (160) comprising:
a plurality of conductive fibres interlaced with a plurality of non-conductive fibres, the conductive fibres defining a plurality of signal paths; and
a plurality of actuators (180) disposed to administer therapeutic steps to a wearer (4) of the first garment (160),
a first computing device (140) comprising:
a first network interface (806);
a first signal interface (808) in signal communication with the plurality of actuators by way of said plurality of signal paths;
at least a first memory (804) storing processor-executable instructions; and
at least a first processor (802) in communication with said at least first memory (804), said at least first processor (802) configured to execute said instructions to:
receive a therapeutic payload data structure (130) by way of said first network interface (806) from a remote therapy controller (110);
process said therapeutic payload data structure (130) to decode a therapeutic signal for actuating at least a given actuator (180) of the plurality of actuators (180), the therapeutic signal reflective of a therapeutic step; and
activate said given actuator (180) using the decoded therapeutic signal to administer said therapeutic step to the wearer (4) of the first garment (160),
wherein the given actuator (180) comprises an electrode for administering functional electrical stimulation, FES, signals to the wearer (4) of said first garment (160) and said therapeutic signal comprises an FES signal, **characterized in that** said electrode comprises conductive fibres interlaced with said non-conductive fibres of said first garment (4), and and wherein said electrode comprises hydrophobic yarn on an outer surface of the garment (160) to maintain wetness longer.

2. The system of claim 1, wherein said first garment (160) further comprises a plurality of sensors (182) disposed to contact the wearer (4) when said first garment (160) is worn.

3. The system of claim 2, wherein said plurality of sensors (182) comprises a sensor for measuring an electrophysiological signal, or
wherein said plurality of sensors (182) comprises a sensor (182) for measuring a biomechanical signal.

4. The system of claim 2, wherein said first processor is further configured to: encode a signal sensed from at least one of the plurality of sensors to generate a feedback data structure, and transmit said feedback data structure to said remote therapy controller.

5. The system of claim 1, wherein said actuator comprises a heater; or
wherein said actuator comprises a drug dispenser, or
wherein said actuator comprises an actuator for imparting mechanical vibration.

6. The system of claim 5, wherein said drug dispenser dispenses a drug transdermally.

7. The system of any of the previous claims, further comprising the remote therapy controller (110) for remote delivery of therapy by way of the first garment (160), the remote therapy controller (110) comprising:
a second control interface (808);
a second network interface (806);
at least a second memory (804) storing processor-executable instructions; and
at least a second processor (802) in communication with the at least second memory (804), the at least second processor (802) configured to execute the processor-executable instructions to:
receive a control signal by way of the second control interface (808);
generate a therapeutic payload data structure (130) upon processing the control signal, the therapeutic payload data structure (130) comprising:
an identifier of a particular actuator (180) disposed at a pre-defined location on said first garment (160), and
control data for actuating said particular actuator (180) to administer a therapeutic signal to the wearer (4) of the first garment (160); and
transmit said therapeutic payload data structure (130) by way of a network (10) to the first computing device (140) in communication with said first garment (160).

8. The system of claim 7, wherein said control data is for actuating said particular actuator (180) for delivering FES signals.

9. The system of claim 7, wherein said at least second processor (802) is further configured to receive a feedback data structure (132) from the first computing device (140) in communication with said first garment by way of said second network interface (806).

10. The system of claim 9, wherein said feedback data structure (132) comprises data encoding:
an electrophysiological signal from the wearer (4) of said first garment; or
a biomechanical signal from the wearer (4) of said first garment.

11. The system of claim 7, wherein the second control interface (808) receives said control signals from a second garment (108) worn by an operator (2) of the remote therapy controller (110).

12. The system of claim 11, wherein said second garment (108) includes an actuator (180), wherein said actuator includes a haptic actuator.

## Patentansprüche

1. System zur entfernten Bereitstellung einer Therapie, wobei das System Folgendes umfasst:
ein erstes Kleidungsstück (160), das Folgendes umfasst:
mehrere leitfähige Fasern, die mit mehreren nicht-leitfähigen Fasern verflochten sind, wobei die leitfähigen Fasern mehrere Signalpfade definieren; und
mehrere Aktuatoren (180), die dazu angeordnet sind, therapeutische Maßnahmen an einem Träger (4) des ersten Kleidungsstücks (160) anzuwenden,
eine erste Rechenvorrichtung (140), die Folgendes umfasst:
eine erste Netzwerkschnittstelle (806);
eine erste Signalschnittstelle (808) in Signalkommunikation mit den mehreren Aktuatoren mittels der mehreren Signalpfade;
wenigstens einen ersten Speicher (804), der prozessorausführbare Anweisungen speichert; und
wenigstens einen ersten Prozessor (802) in Kommunikation mit dem wenigstens ersten Speicher (804), wobei der wenigstens erste Prozessor (802) dazu ausgelegt ist, die Anweisungen zu Folgendem auszuführen:
Empfangen einer Struktur (130) von therapeutischen Nutzdaten mittels der ersten Netzwerkschnittstelle (806) von einer entfernten Therapiesteuerung (110);
Verarbeiten der Struktur (130) von therapeutischen Nutzdaten, um ein therapeutisches Signal zum Betätigen wenigstens eines vorgegebenen Aktuators (180) der mehreren Aktuatoren (180) zu decodieren, wobei das therapeutische Signal eine therapeutische Maßnahme widerspiegelt; und
Aktivieren des vorgegebenen Aktuators (180) unter Verwendung des decodierten therapeutischen Signals, um die therapeutische Maßnahme an dem Träger (4) des ersten Kleidungsstücks (160) anzuwenden,
wobei der vorgegebene Aktuator (180) eine Elektrode zum Anwenden von Signalen für eine funktionelle Elektrostimulation, FES, an dem Träger (4) des ersten Kleidungsstücks (160) umfasst und das therapeutische Signal ein FES-Signal umfasst, **dadurch gekennzeichnet, dass** die Elektrode leitfähige Fasern umfasst, die mit den nicht-leitfähigen Fasern des ersten Kleidungsstücks (4) verflochten sind, und
und wobei die Elektrode ein hydrophobes Garn auf einer Außenfläche des Kleidungsstücks (160) umfasst, um Nässe länger aufrechtzuerhalten.

2. System nach Anspruch 1, wobei das erste Kleidungsstück (160) ferner mehrere Sensoren (182) umfasst, die so angeordnet sind, dass sie mit dem Träger (4) in Kontakt stehen, wenn das erste Kleidungsstück (160) getragen wird.

3. System nach Anspruch 2, wobei die mehreren Sensoren (182) einen Sensor zum Messen eines elektrophysiologischen Signals umfassen, oder
wobei die mehreren Sensoren (182) einen Sensor (182) zum Messen eines biomechanischen Signals umfassen.

4. System nach Anspruch 2, wobei der erste Prozessor ferner zu Folgendem ausgelegt ist: Codieren eines Signals, das von wenigstens einem der mehreren Sensoren erfasst wird, um eine Struktur von Rückkopplungsdaten zu erzeugen, und Übertragen der Struktur von Rückkopplungsdaten an die entfernte Therapiesteuerung.

5. System nach Anspruch 1, wobei der Aktuator eine Heizung umfasst; oder
wobei der Aktuator eine Arzneimittelabgabevorrichtung umfasst, oder
wobei der Aktuator einen Aktuator zum Aufbringen von mechanischer Vibration umfasst.

6. System nach Anspruch 5, wobei die Arzneimittelabgabevorrichtung ein Arzneimittel transdermal abgibt.

7. System nach einem der vorhergehenden Ansprüche, ferner umfassend die entfernte Therapiesteuerung (110) zur entfernten Bereitstellung einer Therapie mittels des ersten Kleidungsstücks (160), wobei die entfernte Therapiesteuerung (110) Folgendes umfasst:
eine zweite Steuerschnittstelle (808);
eine zweite Netzwerkschnittstelle (806);
wenigstens einen zweiten Speicher (804), der prozessorausführbare Anweisungen speichert; und
wenigstens einen zweiten Prozessor (802) in Kommunikation mit dem wenigstens zweiten Speicher (804), wobei der wenigstens zweite Prozessor (802) dazu ausgelegt ist, die prozessorausführbaren Anweisungen zu Folgendem auszuführen:
Empfangen eines Steuersignals mittels der zweiten Steuerschnittstelle (808);
Erzeugen einer Struktur (130) von therapeutischen Nutzdaten bei Verarbeitung des Steuersignals, wobei die Struktur (130) von therapeutischen Nutzdaten Folgendes umfasst:
eine Kennung eines bestimmten Aktuators (180), der an einer vordefinierten Position an dem ersten Kleidungsstück (160) angeordnet ist, und
Steuerdaten zum Betätigen des bestimmten Aktuators (180), um ein therapeutisches Signal an dem Träger (4) des ersten Kleidungsstücks (160) anzuwenden; und
Übertragen der Struktur (130) von therapeutischen Nutzdaten mittels eines Netzwerks (10) an die erste Rechenvorrichtung (140) in Kommunikation mit dem ersten Kleidungsstück (160).

8. System nach Anspruch 7, wobei die Steuerdaten zum Betätigen des bestimmten Aktuators (180) zum Bereitstellen von FES-Signalen dienen.

9. System nach Anspruch 7, wobei der wenigstens zweite Prozessor (802) ferner dazu ausgelegt ist, eine Struktur (132) von Rückkopplungsdaten von der ersten Rechenvorrichtung (140) in Kommunikation mit dem ersten Kleidungsstück mittels der zweiten Netzwerkschnittstelle (806) zu empfangen.

10. System nach Anspruch 9, wobei die Struktur (132) von Rückkopplungsdaten Daten umfasst, die Folgendes codieren:
ein elektrophysiologisches Signal von dem Träger (4) des ersten Kleidungsstücks; oder
ein biomechanisches Signal von dem Träger (4) des ersten Kleidungsstücks.

11. System nach Anspruch 7, wobei die zweite Steuerschnittstelle (808) die Steuersignale von einem zweiten Kleidungsstück (108) empfängt, das von einem Bediener (2) der entfernten Therapiesteuerung (110) getragen wird.

12. System nach Anspruch 11, wobei das zweite Kleidungsstück (108) einen Aktuator (180) beinhaltet, wobei der Aktuator einen haptischen Aktuator beinhaltet.

## Revendications

1. Système pour délivrer une thérapie à distance, le système comprenant :
un premier vêtement (160) comprenant :
une pluralité de fibres conductrices entrelacées avec une pluralité de fibres non conductrices, les fibres conductrices définissant une pluralité de voies de signal ; et
une pluralité d'actionneurs (180) disposés pour administrer des étapes thérapeutiques à un porteur (4) du premier vêtement (160),
un premier dispositif informatique (140) comprenant :
une première interface de réseau (806) ;
une première interface de signal (808) en communication de signal avec la pluralité d'actionneurs par le biais de ladite pluralité de voies de signal ;
au moins une première mémoire (804) stockant des instructions exécutables par processeur ; et
au moins un premier processeur (802) en communication avec ladite au moins une première mémoire (804), ledit au moins un premier processeur (802) étant configuré pour exécuter lesdites instructions pour :
recevoir une structure de données utiles thérapeutiques (130) par le biais de ladite première interface de réseau (806) en provenance d'une unité de commande de thérapie à distance (110) ;
traiter ladite structure de données utiles thérapeutiques (130) pour décoder un signal thérapeutique pour actionner au moins un actionneur donné (180) de la pluralité d'actionneurs (180), le signal thérapeutique reflétant une étape thérapeutique ; et
activer ledit actionneur donné (180) en utilisant le signal thérapeutique décodé pour administrer ladite étape thérapeutique au porteur (4) du premier vêtement (160),
dans lequel l'actionneur donné (180) comprend une électrode pour administrer des signaux d'électrostimulation fonctionnelle, Functional Electrical Stimulation FES, au porteur (4) dudit premier vêtement (160) et ledit signal thérapeutique comprend un signal de FES, **caractérisé en ce que** ladite électrode comprend des fibres conductrices entrelacées avec lesdites fibres non conductrices dudit premier vêtement (4), et
et dans lequel ladite électrode comprend un fil hydrophobe sur une surface extérieure du vêtement (160) pour maintenir une condition mouillée plus longtemps.

2. Système de la revendication 1, dans lequel ledit premier vêtement (160) comprend en outre une pluralité de capteurs (182) disposés pour entrer en contact avec le porteur (4) lorsque ledit premier vêtement (160) est porté.

3. Système de la revendication 2, dans lequel ladite pluralité de capteurs (182) comprend un capteur pour mesurer un signal électrophysiologique, ou
dans lequel ladite pluralité de capteurs (182) comprend un capteur (182) pour mesurer un signal biomécanique.

4. Système de la revendication 2, dans lequel ledit premier processeur est en outre configuré pour : encoder un signal détecté depuis au moins un de la pluralité de capteurs pour générer une structure de données de rétroaction, et transmettre ladite structure de données de rétroaction à ladite unité de commande de thérapie à distance.

5. Système de la revendication 1, dans lequel ledit actionneur comprend un élément chauffant ; ou
dans lequel ledit actionneur comprend un distributeur de médicament, ou
dans lequel ledit actionneur comprend un actionneur pour communiquer une vibration mécanique.

6. Système de la revendication 5, dans lequel ledit distributeur de médicament distribue un médicament de façon transdermique.

7. Système de quelconques des revendications précédentes, comprenant en outre l'unité de commande de thérapie à distance (110) pour délivrer une thérapie à distance par le biais du premier vêtement (160), l'unité de commande de thérapie à distance (110) comprenant :
une seconde interface de commande (808) ;
une seconde interface de réseau (806) ;
au moins une seconde mémoire (804) stockant des instructions exécutables par processeur ; et
au moins un second processeur (802) en communication avec l'au moins une seconde mémoire (804), l'au moins un second processeur (802) étant configuré pour exécuter les instructions exécutables par processeur pour :
recevoir un signal de commande par le biais de la seconde interface de commande (808) ;
générer une structure de données utiles thérapeutiques (130) à la suite du traitement du signal de commande, la structure de données utiles thérapeutiques (130) comprenant :
un identifiant d'un actionneur particulier (180) disposé à un emplacement prédéfini sur ledit premier vêtement (160), et
des données de commande pour actionner ledit actionneur particulier (180) pour administrer un signal thérapeutique au porteur (4) du premier vêtement (160) ; et
transmettre ladite structure de données utiles thérapeutiques (130) par le biais d'un réseau (10) au premier dispositif informatique (140) en communication avec ledit premier vêtement (160).

8. Système de la revendication 7, dans lequel lesdites données de commande sont pour actionner ledit actionneur particulier (180) pour délivrer des signaux de FES.

9. Système de la revendication 7, dans lequel ledit au moins second processeur (802) est en outre configuré pour recevoir une structure de données de rétroaction (132) en provenance du premier dispositif informatique (140) en communication avec ledit premier vêtement par le biais de ladite seconde interface de réseau (806).

10. Système de la revendication 9, dans lequel ladite structure de données de rétroaction (132) comprend des données encodant :
un signal électrophysiologique provenant du porteur (4) dudit premier vêtement ; ou
un signal biomécanique provenant du porteur (4) dudit premier vêtement.

11. Système de la revendication 7, dans lequel la seconde interface de commande (808) reçoit lesdits signaux de commande en provenance d'un second vêtement (108) porté par un opérateur (2) de l'unité de commande de thérapie à distance (110).

12. Système de la revendication 11, dans lequel ledit second vêtement (108) inclut un actionneur (180), dans lequel ledit actionneur inclut un actionneur haptique.
